(19) Europäisches Patentamt
European Patent Office
Office européen des brevets

(11) Veröffentlichungsnummer: **0 086 256**
**B1**

# (12) EUROPÄISCHE PATENTSCHRIFT

(45) Veröffentlichungstag der Patentschrift: **21.08.85**

(51) Int. Cl.⁴: **C 07 C 93/14**, A 61 K 31/135

(21) Anmeldenummer: **82109607.0**

(22) Anmeldetag: **18.10.82**

(54) **1-(2,4-Dichlorphenoxy)-3-(3,4-dimethoxy-Beta-phenethylamino)-2-propanol, Verfahren zu seiner Herstellung und dieses enthaltende pharmazeutische Zusammensetzungen.**

(30) Priorität: **21.10.81 DD 234259**

(43) Veröffentlichungstag der Anmeldung: **24.08.83 Patentblatt 83/34**

(45) Bekanntmachung des Hinweises auf die Patenterteilung: **21.08.85 Patentblatt 85/34**

(84) Benannte Vertragsstaaten: **AT BE CH DE FR GB IT LI NL SE**

(56) Entgegenhaltungen: **Patent Abstracts of Japan**

**Die Akte enthält technische Angaben, die nach dem Eingang der Anmeldung eingereicht wurden und die nicht in dieser Patentschrift enthalten sind.**

(73) Patentinhaber: **VEB Arzneimittelwerk Dresden, Wilhelm-Pieck-Strasse 35, DDR-8122 Radebeul 1 (DD)**

(72) Erfinder: **Bercher, Horst, Dipl.-Chem. Dr. rer. nat., Makarenkostrasse 19 A, DDR-2200 Greifswald (DD)**
Erfinder: **Grisk, Adolf, Prof. Dr. sc. med., Apfelweg 9 A, DDR-2200 Greifswald (DD)**
Erfinder: **Landmann, Hellmut, Dr. sc. med. Dipl.-Bio., Soermusstrasse 1, DDR-8122 Radebeul 1 (DD)**

(74) Vertreter: **Patentanwälte Beetz sen. - Beetz jun. Timpe - Siegfried - Schmitt-Fumian, Steinsdorfstrasse 10, D-8000 München 22 (DE)**

**EP 0 086 256 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft das 1-(2,4-Dichlorphenoxy)-3-(3,4-dimetoxy-$\beta$-phenethylamino)-2-propanol der Formel I, in racemischer und optisch aktiver Form, dessen Ester und Säureadditionssalze, Verfahren zu seiner Herstellung sowie diese Verbindung enthaltende pharmazeutische Zusammensetzungen.

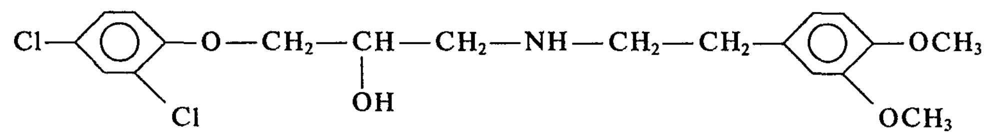 (I)

Die Verbindung I blockiert selektiv die adrenergen $\beta_1$-Rezeptoren bei gleichzeitiger Stimulierung der $\beta_2$-Rezeptoren, wirkt antihypertensiv, antiarrhythmisch und kardioprotektiv und weist zentrale Effekte auf. Sie kann deshalb vorteilhaft zur Therapie und Prophylaxe von Angina pectoris, Myokardinfarkt, Hypertonie, kardialen Arrhythmen, hyperkinetischem Herzsyndrom, Hyperthyreosen, Migräne, Parkinsonismus u. a. eingesetzt werden.

1-Phenoxy-3-alkylamino-2-propanole sind mehrfach beschrieben worden (DE-PS 2 259 489, DE-PS 2 213 044, DD-PS 45 360, BE-PS 783 086, NL-PS 6 813 616, GB-PS 1 148 563, NL-PS 7 307 821, DD-PS 93 349, GB-PS 1 078 852, BE-PS 746 107). Auch das 1-(2,5-Dichlorphenoxy)-3-(3,4-dimethoxy-$\beta$-phenethylamino)-2-propanol ist bekannt (JA-OS 52 053 829); es unterscheidet sich in seiner Wirksamkeit gegenüber der erfindungsgemäßen 2,4-Dichlor-Verbindung jedoch darin, daß die ausgeprägten stimulierenden Effekte an den $\beta$-Rezeptoren der Gefäße, die eine große Bedeutung für den therapeutischen Einsatz besitzen, fehlen.

Aufgabe der Erfindung ist es, einen neuen $\beta$-adrenolytischen Wirkstoff vom Phenoxypropanolamin-Typ bereitzustellen, der mehrere vorteilhafte pharmakologische Eigenschaften in bisher unbekannter Kombination in sich vereint, Verfahren zu seiner Herstellung sowie ihre pharmazeutische Verwendung anzugeben.

Die Aufgabe wird anspruchsgemäß gelöst. Die Verbindung I wird als Racemat oder in optisch aktiver Form hergestellt und kann in Form ihrer Ester und Säureadditionssalze vorliegen.

Die neue Verbindung der Formel I zeichnet sich durch überlegene pharmakologische Eigenschaften aus, da sie spezifisch die $\beta_1$-Rezeptoren blockiert und die $\beta_2$-Rezeptoren stimuliert. Damit werden in bisher unbekannter Weise zwei antianginöse Wirkprinzipien vereint, nämlich Senkung des Sauerstoffverbrauchs des Herzens und Gefäßerweiterung und damit Senkung des peripheren Gefäßwiderstandes und Herzentlastung. Hinzu tritt ein vielfach erwünschter bronchodilatorischer Effekt.

Die erfindungsgemäße Verbindung I kann auf folgenden Wegen hergestellt werden:

a) Umsetzung von 1-(2,4-Dichlor-phenoxy)-3-chlor-2-propanol mit 3,4-Dimethoxy-$\beta$-phenethylamin. Um eine möglichst vollständige Umsetzung zu erhalten, wird die Reaktion in Gegenwart einer Base durchgeführt, vorzugsweise in Gegenwart eines Überschusses des Reaktionsteilnehmers 3,4-Dimethoxy-$\beta$-phenethylamin. Einige Beispiele für geeignete Basen sind Alkalihydroxide, wie Kalium- und Natriumhydroxid, Alkalicarbonate, wie Kalium- und Natriumcarbonat, und tertiäre Amine, wie Triethylamin und Tripopylamin. Die Umsetzung der Reaktionsteilnehmer wird zweckmäßig in Gegenwart eines organischen Lösungsmittels durchgeführt. Beispiele für geeignete Lösungsmittel sind Alkanole, wie Ethanol und 2-Propanol, Ether, wie Dioxan, Ketone, wie Aceton und Methylethylketon, Kohlenwasserstoffe, wie Benzol, Toluol und Xylol und chlorierte Kohlenwasserstoffe, wie Chloroform, Tetrachlormethan und Tetrachlorethan. Die Reaktionszeiten und -temperaturen können weitgehend variiert werden. Die Umsetzung wird allgemein innerhalb von 1 bis 43 h bei Temperaturen von 20 bis 200°C durchgeführt. Das Reaktionsprodukt wird direkt als freie Base oder nach Behandlung mit einer Säure als Säureadditionssalz isoliert. Vorzugsweise wird das Halogenhydrin mit der zweifachen äquimolaren Menge 3,4-Dimethoxy-$\beta$-phenethylamin in 2-Propanol gelöst und 16 h lang am Rückfluß gekocht, worauf das 2-Propanol im Vakuum abdestilliert wird. Das Produkt wird mit einem organischen Lösungsmittel extrahiert, vorzugsweise mit einem Lösungsmittel, das das gebildete 3,4-Dimethoxy-$\beta$-phenethylaminhydrochlorid nicht löst. Beispiele für geeignete Lösungsmittel sind Aceton und Ethylacetat. Die Umsetzung kann auch in einem mit Wasser nicht bzw. wenig mischbaren organischen Lösungsmittel, wie z. B. Chloroform, erfolgen und das gebildete 3,4-Dimethoxy-$\beta$-phenethylaminhydrochlorid durch Extraktion mit Wasser aus dem Reaktionsgemisch abgetrennt werden.
Das Endprodukt wird aus dem Reaktionsgemisch durch Behandlung mit Chlorwasserstofflösung im 2-Propanol oder Ether oder durch Einleiten von wasserfreiem Chlorwasserstoff als Hydrochlorid isoliert.

b) Umsetzung von 1-(2,4-Dichlorphenoxy)-2,3-epoxypropan mit 3,4-Dimethoxy-$\beta$-phenethylamin. Die Reaktion erfolgt gegebenenfalls in einem Lösungsmittel und bei erhöhter Temperatur. Im allgemeinen wird die Umsetzung innerhalb von 15 min bis 48 h bei einer Temperatur von 15 bis 150°C durchgeführt. Beispiele für geeignete Lösungsmittel sind Alkanole, wie Methanol, Ethanol und 2-Propanol, Ether, wie Dioxin, Ketone, wie Aceton und Methylethylketon, Kohlenwasserstoffe,

wie Benzol, Toluol und Xylol und chlorierte Kohlenwasserstoffe, wie Chloroform, Tetrachlormethan und Tetrachlorethan. Die Reaktionsteilnehmer werden im allgemeinen in äquimolaren Mengen umgesetzt, obwohl sie auch in mäßigem Überschuß eingesetzt werden können.

c) Hydrolyse von 3-(3,4-Dimethoxy-$\beta$-phenethyl)-5-(2,4-dichlorphenoxymethyl)-2-oxazolidin.
Die Hydrolyse des Oxazolidins führt man zweckmäßigerweise mit einer Alkalilauge, wie z. B. mit wäßrigen Lösungen von Natrium- oder Kaliumhydroxid, durch. Gegebenenfalls kann bei dieser Umsetzung auch ein organisches Lösungsmittel verwendet werden. Geeignete Lösungsmittel sind z. B. Alkanole, wie Ethanol und Methanol.

d) Umsetzung von 2,4-Dichlorphenol mit 1,2-Epoxy-3-(3,4-dimethoxy-$\beta$-phenethylamino)-propan oder

e) Umsetzung von 2,4-Dichlorphenol mit einem 1-Halogen-3-(3,4-dimethoxy-$\beta$-phenethylamino)-2-propanol, vorzugsweise dem 1-Chlor- bzw. 1-Brom-3-(3,4-dimethoxy-$\beta$-phenethylamino)-2-propanol.
Die Umsetzungen d) und e) können zweckmäßig in Gegenwart eines säurebindenden Mittels, z. B. Kalium- oder Natriumhydroxid, durchgeführt werden. Andererseits kann ein entsprechendes Alkaliphenolat als Ausgangsstoff verwendet werden. Die Umsetzungen können gegebenenfalls in einem Lösungsmittel und bei erhöhter Temperatur, z. B. der Siedetemperatur des Lösungsmittels, erfolgen. Beispiele für geeignete Lösungsmittel sind Alkanole, wie Ethanol und 2-Propanol, Kohlenwasserstoffe, wie Benzol, Toluol und Xylol und chlorierte Kohlenwasserstoffe, wie Chloroform, Tetrachlormethan und Tetrachlorethan.

f) Umsetzung von 1-(2,4-Dichlorphenoxy)-3-amino-2-propanol mit 3,4-Dimethoxy-$\beta$-phenethylchlorid. Die Reaktion wird zweckmäßig in Gegenwart eines säurebindenden Mittels, z. B. Natriumhydroxid oder Natriumhydrogencarbonat, durchgeführt. Die Umsetzung kann gegebenenfalls bei erhöhter Temperatur und in einem Lösungsmittel, z. B. einem Alkanol, wie Ethanol, n-Propanol und 2-Propanol, erfolgen.

g) Umsetzung 2,4-Dichlorphenol mit 1-(3,4-Dimethoxy-$\beta$-phenethyl)-3-azetidinol. Die Reaktion wird zweckmäßig unter Ausschluß von Wasser sowie Sauerstoff und in Gegenwart eines basischen oder sauren Katalysators, wie Natrium- oder Kaliumhydroxid, Triethylamin und Trifluoressigsäure, durchgeführt. Die Umsetzung kann gegebenenfalls bei erhöhter Temperatur, vorzugsweise bei 120 bis 250° C, und in einem Lösungsmittel erfolgen. Geeignete Lösungsmittel sind z. B. Alkanole, wie Benzylalkohol und chlorierte Kohlenwasserstoffe, wie Chlorbenzol.

h) Abspaltung einer an der alkoholischen Hydroxylgruppe der Seitenkette der Verbindung I befindlichen Schutzgruppe. Geeignete Schutzgruppen sind z. B. Acylgruppen, wie die Acetyl-, Benzoyl- und Hexanoylgruppe, oder Acetalgruppen, wie die Tetrahydropyranylgruppe. Die Abspaltung erfolgt zweckmäßigerweise durch Erwärmen mit wäßriger oder alkanolischer Alkalilauge oder vorzugsweise bei Acetalen mit einer Säure, wie z. B. Salzsäure.

Die für die Durchführung der Verfahren a bis g benötigten Ausgangssubstanzen sind z. T. bereits bekannt, z. T. können sie nach üblichen Verfahren hergestellt werden. Die erfindungsgemäße Verbindung besitzt ein asymmetrisches Kohlenstoffatom an der -CH(OH)-Gruppe und kann daher als Racemat wie auch in Form der optischen Antipoden vorliegen. Letztere können in an sich bekannter Weise außer durch Einsetzen von optisch aktivem Ausgangsmaterial auch durch Racematspaltung mit üblichen Hilfssäuren, wie z. B. der (+)- oder (—)-Form der Weinsäure, der Dibenzoylweinsäure, der Mandelsäure, der 3-Bromcampher-8-sulfonsäure und der Campher-10-sulfonsäure, erhalten werden.

Die erfindungsgemäße Verbindung I kann in üblicher Weise in ihre physiologisch verträglichen Säureadditionssalze und Ester übergeführt werden. Beispiele für geeignete Säuren zur Gewinnung der Säureadditionssalze sind Salzsäure, Bromwasserstoffsäure, Schwefelsäure, Phosphorsäure, Essigsäure, Oxalsäure, Methansulfonsäure, Milchsäure, Weinsäure, Apfelsäure und Maleinsäure. Die Überführung der erfindungsgemäßen Verbindung in ihre Ester kann z. B. durch Umsetzung mit Acylhalogeniden oder Acylanhydriden, wie z. B. Acetylchlorid, Acetanhydrid und Butyrylchlorid, erfolgen.

Die erfindungsgemäße Verbindung I zeigt überraschenderweise im Tierversuch gegenüber den bekannten $\beta$-Rezeptorenblockern Propranolol (Obsidan®), Talinolol (Cordanum®), Practolol und Cl 775 überlegene pharmakologische Eigenschaften. Beispielsweise vereint I in einer für $\beta$-Rezeptorenblokker bisher unbekannten Kombination zwei antianginöse Wirkungsprinzipien:

— Durch Blockierung der kardialen $\beta_1$-Rezeptoren wird der Sauerstoffverbrauch des Herzens gesenkt;
— durch Stimulation der adrenergen $\beta_2$-Rezeptoren werden die Gefäße dilatiert, der periphere Gefäßwiderstand gesenkt und somit das Herz entlastet.

Am Myokardinfarktmodell der Ratte (orale Applikation von 3,6 und 12 mg/kg I über 28 Tage vor Ligatur der A. coronaria sinistra, Bewertungskriterien: CPK, GOT, EKG-Veränderungen, relatives Infarktgewicht) zeigte I nach prophylaktischer Gabe eine etwa zweimal stärkere kardioprotektive Wirkung als bei Propranolol. Die Stärke der $\beta_1$-Rezeptorenblockierung von I ist speciesabhängig (Isoproterenolantagonismus, Propranolol = 100):

| Testmodell | Verbindung I | Talinolol |
|---|---|---|
| Ratte | | |
| Herzfrequenz (Ganztier) | 340 | 60 |
| Kontraktilität (isol. Vorhof) | 24 | 21 |
| Lipolyse (Epididymalfett) | 220 | 96 |
| Meerschweinchen | | |
| Kontraktilität (isol. Vorhof) | 76 | 80 |
| Kaninchen | | |
| Darmperistaltik | 100 | 35 |

An den $\beta_1$-Rezeptoren von Katze und Hund (Herzfrequenz und Kontraktilität am Ganztier) war die Verbindung I nur halb so wirksam wie Propranolol. Während Propranolol den blutdrucksenkenden Effekt des Isoproterenols an Katze und Hund durch Blockierung der $\beta_2$-Rezeptoren der Gefäße aufhebt, kommt es nach Gabe von I durch die $\beta_2$-adrenerge Stimulierung zu einer Verstärkung der Isoproterenolwirkung. Die durch die $\beta_2$-adrenerge Wirkung der erfindungsgemäßen Verbindung bedingte Vasodilatation führt zu einem für die bekannten $\beta$-Rezeptorenblocker ungewöhnlich starken antihypertensiven Effekt, der etwa 10 mal stärker ist als beim Propranolol (spontan hypertensive Ratte). Von großer therapeutischer Bedeutung ist auch die Stimulation der adrenergen $\beta_2$-Rezeptoren der Bronchialmuskulatur, die sich sowohl am isolierten Trachealmuskel als auch im Ganztierversuch nachweisen läßt. So führt die Gabe von 0,1 mg/kg der erfindungsgemäßen Verbindung am narkotisierten Hund zu einer isoproterenolähnlichen Vergrößerung des Atemzeitvolumens, während Propranolol dieses durch seine bronchokonstriktorischen Effekte verkleinert. Am wachen Meerschweinchen bewirken 0,76 mg/kg eine Verdopplung der Toleranzzeit gegenüber dem asthmaauslösenden Histamin (Isoproterenol: $ED_{50} = 0,04$ mg/kg), während 9,1 mg/kg Propranolol diese als Ausdruck der bronchokonstriktorischen Wirkung um die Hälfte herabsetzt. Aus diesem Grunde ist die Anwendung von nichtselektiv wirkenden $\beta$-Rezeptorenblockern bei Patienten mit obstruktiven Atemwegserkrankungen kontraindiziert. Aber auch die Anwendung der sogenannten kardioselektiven $\beta$-Rezeptorenblocker, z. B. von Practolol, Talinolol und Cl 775, ist bei diesen Patienten, wie die klinischen Erfahrungen zeigen, bedenklich, da sie bei entsprechender Dosierung bzw. Disposition des Patienten zur Dyspnoe führen bzw. einen Asthmaanfall auslösen können. Da die Verbindung I $\beta_2$-adrenerge Wirkungen aufweist, kann sie vorteilhaft ohne die Gefahr einer Erhöhung des Atemwegwiderstandes oder der Auslösung von Asthma-Anfällen verwendet werden. Daneben zeigt I ausgeprägte und therapeutisch bedeutsame antiarythmische Effekte, die die von Propranolol, Talinolol, Practolol und dem bekannten Antiarrythmikum Tachmalin übertreffen ($ED_{50}$, Erhöhung der elektrischen Flimmerschwelle am Meerschweinchen: I = 0,22 mg/kg, Propranolol = 1,4 mg/kg, Talinolol = 0,31 mg/kg, Tachmalin = 0,71 mg/kg). Auch die zentralen Wirkungen von I sind therapeutisch bedeutsam; so zeigte I am Nikotintremor des Meerschweinchens etwa die gleiche Antiparkinsonwirkung wie Trihexylphenidyl (Parkopan®). Die Toxizität von I ist ferner geringer als die von Propranolol und Talinolol (i. p.-Applikation an der Maus, $LD_{50}$: I = 141 mg/kg, Propanolol 107 mg/kg, Talinol 96 mg/kg).

Die erfindungsgemäße Verbindung besitzt die gleichen Indikationsgebiete wie die schon bekannten $\beta$-Rezeptorenblocker, weist jedoch durch die bisher neuartige Kombination von $\beta_1$-Rezeptorenblockierung bei gleichzeitiger $\beta_2$-Stimulierung stärkere antianginöse, kardioprotektive und anthypertensive Effekte auf und kann zusätzlich auch bei Patienten mit obstruktiven Atemwegserkrankungen angewandt werden. Sie kann daher vorteilhaft zur Behandlung von Angina pectoris, Myokardinfarkt, Hypertonie und zur Therapie zentralnervöser Erkrankungen, wie z. B. Parkinsonismus, Migräne, Drogensucht und Psychosen, eingesetzt werden.

Die therapeutische Einzeldosis der erfindungsgemäßen Verbindung liegt bei intravenöser Applikation zwischen 1 und 20 mg, bei oraler Gabe zwischen 10 und 100 mg.

Die erfindungsgemäße Verbindung kann in die üblichen galenischen Anwendungsformen, wie Tabletten, Kapseln, Dragees, Pulver, Lösungen, Emulsionen und Depotformen, gebracht werden, wobei zu deren Herstellung die üblichen pharmazeutischen Hilfsstoffe sowie die üblichen Fertigungsmethoden benutzt werden können. Die erfindungsgemäßen Verbindungen lassen sich vorteilhaft in Kombination mit anderen pharmkodynamisch wirksamen Stoffen anwenden. Beispiele für zur Kombination geeignete Stoffe sind antianginöse Pharmaka, wie Propantrioltrinitrat, Pentaerythrityltetranitrat und Dipyridamol, Herzglykoside, wie Digitoxin und Digoxin, Antihypertensiva, wie Clonidin und Dihydralazin, Diuretika, wie Furosemid und Hydrochlorothiazid, und zentral wirksame Pharmaka, wie Diazepam und Lepinal.

Die Erfindung wird im folgenden anhand einiger Ausführungsbeispiele näher erläutert.

Beispiel 1

5,1 g 1-(2,4-Dichlorphenoxy)-3-chlor-2-propanol und 7,2 g 3,4-Dimethoxy-β-phenethylamin werden in 15 ml Ethylacetat gelöst und 20 h am Rückfluß erhitzt. Das ausgefallene 3,4-Dimethoxy-β-phenethylamin-hydrochlorid wird abgesaugt, das Reaktionsgemisch mit Wasser extrahiert und die organische Phase über Natriumsulfat getrocknet. Der nach dem Eindampfen erhaltene Rückstand wird in überschüssiger Chlorwasserstofflösung in 2-Propanol gelöst. Das Reaktionsgemisch wird eingedampft, der Rückstand mit Ether gewaschen und aus Ethylacetat/Ethanol umkristalliert. Man erhält das Hydrochlorid des 1-(2,4-Dichlorphenoxy)-3-(3,4-dimethoxy-β-phenethylamino)-2-propanols als farblose Kristalle vom F. 151 bis 152° C. Ausbeute 6,5 g (74% d. Th.).

Beispiel 2

21,9 g 1-(2,4-Dichlorphenoxy)-2,3-epoxypropan werden in 50 ml 2-Propanol gelöst, mit 18,1 g 3,4-Dimethoxy-β-phenethylamin in 50 ml 2-Propanol versetzt und 10 h am Rückfluß erhitzt. Man destilliert dann das 2-Propanol im Vakuum ab, nimmt den Rückstand in Ether auf und wäscht die etherische Lösung mit Wasser. Die etherische Lösung wird über Natriumsulfat getrocknet, abfiltriert und eingedampft. Man erhält das 1-(2,4-Dichlorphenoxy)-3-(3,4-dimethoxy-β-phenethylamino)-2-propanol als farblosen, kristallinen Rückstand der aus Ethylacetat/Ethanol umkristallisiert wird. Hierbei fällt die Verbindung als farbloses, feinkristallines Pulver an; F. 120 bis 121° C. Ausbeute 38 g (95% d. Th.).

Beispiel 3

5 g (3,4-Dimethoxy-β-phenethyl)-5-(2,4-dichlorphenoxy-methyl)-2-oxazolidinon werden in 20 ml Methanol gelöst und mit 20 ml 50%iger Natronlauge 3 h am Rückfluß erhitzt. Das Methanol wird abdestilliert, worauf mit Salzsäure angesäuert wird. Anschließend wird mit Ether extrahiert und die wäßrige Phase mit Natronlauge alkalisch gemacht. Das ausgefallene 1-(2,4-Dichlorphenoxy)-3-(3,4-dimethoxy-β-phenethylamino)-2-propanol wird abgesaugt und aus Ethylacetat/Ethanol umkristallisiert. Man erhält ein farbloses, feinkristallines Pulver; F. 120 bis 121° C. Ausbeute 2,8 g (60% d. Th.).

Beispiel 4

16,3 g 2,4-Dichlorphenol und 4,0 g Natriumhydroxid werden in 300 ml Ethanol gelöst; nach Zusatz von 28,5 g 1,2-Epoxy-3-(3,4-dimethoxy-β-phenethylamino)-propan wird das Reaktionsgemisch 4 h am Rückfluß erhitzt. Danach wird das Gemisch im Vakuum zur Trockne eingedampft; der Rückstand wird in 80 ml Chloroform aufgenommen und mit Wasser extrahiert. Die organische Phase wird über Natriumsulfat getrocknet. Nach dem Abfiltrieren wird die doppelte Menge Ether der Lösung zugesetzt und trockener Chlorwasserstoff eingeleitet. Der gebildete Niederschlag wird abgesaugt, mit Ether gewaschen und aus verdünntem Ethanol umkristallisiert. Man erhält das Hydrochlorid des 1-(2,4-Dichlorphenoxy)-3-(3,4-dimethoxy-β-phenethylamino)-2-propanols als farbloses, feinkristallines Pulver. F. 151 bis 152° C. Ausbeute 9,7 g (22% d. Th.).

Beispiel 5

16,3 g 2,4-Dichlorphenol und 8,0 g Natriumhydroxid werden in 100 ml Ethanol und 20 ml Wasser gelöst und mit 32,9 g 1-Chlor-3-(3,4-dimethoxy-β-phenethylamino)-2-propanol-hydrochlorid versetzt. Die Mischung wird 4 h unter Rückfluß erhitzt und im Vakuum zur Trockne eingedampft. Zur Gewinnung des 1-(2,4-Dichlorphenoxy)-3-(3,4-dimethoxy-β-phenethylamino)-2-propanols wurde der Rückstand, wie unter Beispiel 2 beschrieben, weiterverbessert. Ausbeute 11,2 g (28% d. Th.).

Beispiel 6

Ein Gemisch von 27,3 g 1-(2,4-Dichlorphenoxy)-3-amino-2-propanol, 16,8 g Natriumhydrogencarbonat, 20,1 g 3,4-Dimethoxy-β-phenethylchlorid und 300 ml n-Propanol wird 18 h unter Rückfluß erhitzt, danach im Vakuum bis zur Trockne eingedampft. Der Rückstand wird wie in Beispiel 2 aufgearbeitet. Ausbeute 24,3 g (56% d. Th.).

Beispiel 7

Eine Mischung aus 13,1 g 2,4-Dichlorphenol, 23,7 g 1-(3,4-Dimethoxy-β-phenethyl)-3-azetidinol und 0,5 g Kaliumhydroxid in 50 ml Benzylalkohol wird unter Rühren im Stickstoffstrom 15 h auf 140°C erhitzt. Nach dem Abkühlen werden 150 ml Ethylacetat zugegeben, worauf dreimal mit je 100 ml 2 n Salzsäure ausgeschüttelt wird. Die wäßrige Phase wird mit Ethylacetat gewaschen. Nach dem Trocknen der organischen Phase über Natriumsulfat wird abgesaugt und trockener Chlorwasserstoff eingeleitet. Das ausgefallene Hydrochlorid des 1-(2,4-Dichlorphenoxy)-3-(3,4-dimethoxy-β-phenethylamino)-2-propanols wird abgesaugt und aus Ethylacetat/Ethanol umkristallisiert. Man erhält ein farbloses, feinkristallines Pulver. F. 151 bis 152°C. Ausbeute 12,4 g (36% d. Th.).

Beispiel 8

3 g 1-(2,4-Dichlorphenoxy)-3-(3,4-dimethoxy-β-phenethyl)-aminopropan-tetrahydropyranylether-oxalat werden in 25 ml 2 n Salzsäure gelöst und 15 min unter Rückfluß erhitzt. Das Reaktionsgemisch wird mit Ether extrahiert, wonach die wäßrige Phase mit Natronlauge alkalisch gemacht wird. Das abgeschiedene Öl wird in Ether aufgenommen; die etherische Lösung wird mit Wasser gewaschen, über Natriumsulfat getrocknet, abfiltriert und auf etwa die Hälfte eingeengt. Nach dem Einleiten von trockenem Chlorwasserstoff bildet sich ein Niederschlag, der abgesaugt, mit Ether gewaschen und aus Wasser umkristallisiert wird. Man erhält das Hydrochlorid des 1-(2,4-Dichlorphenoxy)-3-(3,4-dimethoxy-β-phenethylamino)-2-propanols als farbloses, feinkristallines Pulver. F. 151 bis 152°C. Ausbeute 1,7 g (62% d. Th.).

**Patentansprüche**

1. 1-(2,4-Dichlorphenoxy)-3-(3,4-dimethoxy-β-phenethylamino)-2-propanol der Formel I

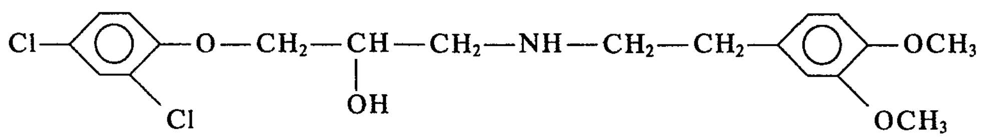

(I)

als Racemat oder in optisch aktiver Form.

2. Verbindung der Formel I nach Anspruch 1 in Form ihrer Ester und Säureadditionssalze.

3. Verfahren zur Herstellung der Verbindung der Formel I nach Anspruch 1 oder 2, gekennzeichnet durch

a) Umsetzung von 1-(2,4-Dichlorphenoxy)-3-chlor-2-propanol mit 3,4-Dimethoxy-β-phenethylamin oder
b) Umsetzung von 1-(2,4-Dichlorphenoxy)-2,3-epoxy-propan mit 3,4-Dimethoxy-β-phenethylamin oder
c) Hydrolyse von 3-(3,4-Dimethoxy-β-phenethyl)-5-(2,4-dichlorphenoxymethyl)-2-oxazolidinon oder
d) Umsetzung von 2,4-Dichlorphenoxy mit 1,2-Epoxy-3-(3,4-dimethoxy-β-phenethylamino)-propan oder
e) Umsetzung von 2,4-Dichlorphenol mit einem 1-Halogen-3-(3,4-dimethoxy-β-phenethylamino)-2-propanol oder
f) Umsetzung von 1-(2,4-Dichlorphenoxy)-3-amino-2-propanol mit 3,4-Dimethoxy-β-phenethylchlorid oder
g) Umsetzung von 2,4-Dichlorphenol mit 1-(3,4-Dimethoxy-β-phenethylamin)-3-azetidinol oder
h) Abspaltung einer an der alkoholischen Hydroxylgruppe der Seitenkette der Verbindung der Formel I befindlichen Schutzgruppe

und gegebenenfalls anschließende Überführung der nach a) bis h) erhaltenen Verbindungen durch Umsetzung mit geeigneten Hilfssäuren in die entsprechenden diastereomeren Salze und deren Auftrennung.

4. Verfahren nach Anspruch 3, dadurch gekennzeichnet, daß optisch aktive Ausgangsstoffe eingesetzt werden.

5. Pharmazeutische Zusammensetzungen, gekennzeichnet durch einen Gehalt an der Verbindung der Formel I nach Anspruch 1 oder 2.

6. Arzneimittel mit einem antianginösen, antiarrhythmischen, antihypertensiven, kardioprotektiven und bronchodilatierenden Wirkungsprofil, gekennzeichnet durch einen Wirkstoffgehalt an Verbindung I nach Anspruch 1 oder 2 von 1 bis 100 mg pro Dosierungseinheit.

## Claims

1. 1-(2,4-Dichlorophenoxy)-3-(3,4-dimethoxy-β-phenethylamino)-2-propanol of formula I

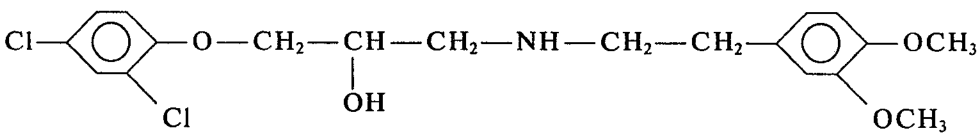

(I)

as racemate or in optically active form.

2. A compound of formula I according to claim 1 in the form of its esters and acid addition salts.

3. A process for preparing the compound of formula I according to claim 1 or 2, characterized by

a) reaction of 1-(2,4-dichlorophenoxy)-3-chloro-2-propanol with 3,4-dimethoxy-β-phenethylamine, or
b) reaction of 1-(2,4-dichlorophenoxy)-2,3-epoxypropane with 3,4-dimethoxy-β-phenethylamine, or
c) hydrolysis of 3-(3,4-dimethoxy-β-phenethyl)-5-(2,4-dichlorophenoxymethyl)-2-oxazolidinone, or
d) reaction of 2,4-dichlorophenol with 1,2-epoxy-3-(3,4-dimethoxy-β-phenethylamino)propane, or
e) reaction of 2,4-dichlorophenol with a 1-halo-3-(3,4-dimethoxy-β-phenethylamino)-2-propanol, or
f) reaction of 1-(2,4-dichlorophenoxy)-3-amino-2-propanol with 3,4-dimethoxy-β-phenethyl chloride, or
g) reaction of 2,4-dichlorophenol with 1-(3,4-dimethoxy-β-phenethyl)-3-azetidinol, or
h) cleavage of a protective group positioned at the alcoholic hydroxyl group of the side chain in the compound of formula I,

and optionally subsequent conversion of the compounds obtained according to a) to h) by reaction with suitable auxiliary acids to the corresponding diastereometric salts, and separation of the latter.

4. Process according to claim 3, characterized by the use of optically active starting materials.

5. Pharmaceutical compositions characterized by a content of the compound of formula I according to claim 1 or 2.

6. Pharmaceutical preparation with antianginous, antiarrhythmic, antihypertensive, cardioprotective, and bronchodilatoric activity profile, characterized by a content of compound I according to claim 1 or 2 of from 1 to 100 milligram per dosage unit, as active ingredient.

## Revendications

1. (2,4-dichlorophénoxy)-3-(3,4-diméthoxy-β-phénéthylamino)-2-propanol de formule I

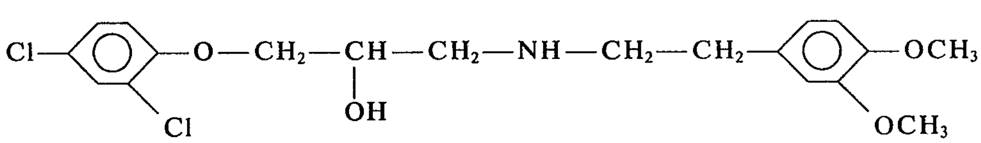

(I)

sous forme racémique ou sous forme optiquement active.

2. Composé de formule I selon la revendication 1 sous forme d'esters et de sels d'addition acides.

3. Procédé de production du composé de formule I selon la revendication 1 ou 2, caractérisé par:

a) la transformation de 1-(2,4-dichlorophénoxy)-3-chlore-2-propanol à l'aide de 3,4-diméthoxy-β-phénéthylamine ou
b) la transformation de 1-(2,4-dichlorophénoxy)-2,3-époxypropane à l'aide de 3,4-diméthoxy-β-phénéthylamine ou
c) l'hydrolyse de 3-(3,4-diméthoxy-β-phénéthyl)-5-(2,4-dichlorophénoxyméthyle)-2-oxazolidinone ou
d) la transformation de 2,4-dichlorophénol à l'aide de 1,2-époxy-3-(3,4-diméthoxy-β-phénéthylamino)-propane ou
e) la transformation de 2,4-dichlorophénol à l'aide d'un 1-halogéne-3-(3,4)-diméthoxy-β-phénéthylamino)-2-propanol ou
f) la transformation de 1-(2,4-dichlorophénoxy)-3-amino-2-propanol à l'aide de 3,4 diméthoxy-β-chlorure de phénéthyl ou
g) la transformation de 2,4-dichlorophénol à l'aide de 1-(3,4-diméthoxy-β-phénéthyl)-3-acétidinol ou
h) l'élimination d'un groupe protecteur situé sur le groupe hydroxyle alcoolique de la chaîne latérale du composé de la formule I

et ensuite, le cas échéant, la transformation des composés obtenus par a) à h) par transformation à

l'aide d'acides auxiliaires adéquats en sels diastéréo-isomères correspondants et séparation de ceux-ci.

4. Procédé selon la revendication 3, caractérisé en ce que des substances de base optiquement actives sont employées.

5. Composition pharmaceutique caractérisé par une teneur en composé de formule I selon l'une des revendications 1 ou 2.

6. Médicament à spectre d'action anti-angineuse, anti-arythmique, anti-hypertensive, cardio-protectrice et broncho-dilatatrice, caractérisé par une teneur en produit actif du composé I selon l'une des revendications 1 ou 2 de 1 à 100 mg par unité de dosage.